(19) Europäisches Patentamt
European Patent Office
Office européen des brevets

(11) **EP 3 416 078 A1**

(12) **EUROPEAN PATENT APPLICATION**

(43) Date of publication:
**19.12.2018 Bulletin 2018/51**

(51) Int Cl.:
*G06F 21/32* (2013.01)          *H04L 29/06* (2006.01)
*A61B 5/053* (2006.01)          *A61B 5/00* (2006.01)
*G06F 21/34* (2013.01)

(21) Application number: **17176386.5**

(22) Date of filing: **16.06.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Stichting IMEC Nederland**
**5656 AE  Eindhoven (NL)**

(72) Inventors:
• **LEE, Seulki**
  **3001 Leuven (BE)**
• **DE FRANCISCO MARTIN, Ruben**
  **3001 Leuven (BE)**

(74) Representative: **AWA Sweden AB**
**P.O. Box 5117**
**200 71 Malmö (SE)**

(54) **A BIOMETRIC IDENTIFICATION SYSTEM**

(57)    According to an aspect of the present inventive concept there is provided a biometric identification system comprising:

a bio-impedance measurement unit adapted to be worn by a user, the bio-impedance measurement unit including:

a plurality of measurement electrodes;

a measurement circuit adapted to perform bio-impedance measurements on the user via a plurality of different groups of said plurality of measurement electrodes, each of said groups including at least two measurement electrodes,

wherein the impedance measurement circuit, for each group of measurement electrodes, is adapted to measure an impedance in each frequency channel of a set of frequency channels,

the system further comprising:

a storage unit adapted to store each measured impedance in a measurement data set, and

an identification unit adapted to perform a comparison based on the measurement data set and a reference data set.

Fig. 2

EP 3 416 078 A1

## Description

Technical field

The present inventive concept relates to a biometric identification system.

Background

In an era with an ever-increasing number of electronics devices and services, secure user authentication is an area of increasing interest and development.

User identification based on biometric data represents a both secure and convenient alternative to more traditional methods, such as those based on passwords or codes. Biometric identification may herein be used to refer to both identification and authentication.

It is known to implement biometric identification technology in electronic devices such as desktop computers, laptops or other portable electronics such as tablet computing devices and mobile phones. Examples include facial recognition and finger print recognition. However, such known technology typically requires a user to interact with a user interface of the electronic device to initiate the identification method and/or to physically interact with the device in a manner dictated by the identification method, such as by positioning himself in front of a camera of the device to enable facial recognition, or to position his finger on a sensor of the device to enable finger print recognition.

It would be desirable to provide an identification system based on biometrics enabling a reliable yet convenient identification.

Summary of the inventive concept

An objective of the present inventive concept is to address this need. Additional and alternative objectives may be understood from the following.

According to an aspect of the present inventive concept there is provided a biometric identification system comprising:

a bio-impedance measurement unit adapted to be worn by a user, the bio-impedance measurement unit including:

a plurality of measurement electrodes;
a measurement circuit adapted to perform bio-impedance measurements on the user via a plurality of different groups of said plurality of measurement electrodes, each of said groups including at least two measurement electrodes,
wherein the impedance measurement circuit, for each group of measurement electrodes, is adapted to measure an impedance in each frequency channel of a set of frequency channels,

the system further comprising:

a storage unit adapted to store each measured impedance in a measurement data set, and

an identification unit adapted to perform a comparison based on the measurement data set and a reference data set.

The inventive system enables collection of biometric data based on bio-impedance measurements on the user / wearer of the bio-impedance measurement unit.

As realized by the inventors, bio-impedance measurements on corresponding body parts (such as a wrist, an ankle, a chest portion) of different people typically yield characteristic and repeatable results. Thus, by measuring bio-impedance via a plurality of measurement electrodes, i.e. at a plurality of different positions of the user, in accordance with the inventive system, a measurement data set may be determined which forms a biometric for the user.

By performing a comparison based on the measurement data set and a reference data set, the system may accordingly determine whether the measurement data set determined for the current user of the system corresponds to or matches the reference data set or not. A result of the comparison may hence serve as a basis for identifying whether a user is a valid user of the wearable and/or of another device or service in communication with the identification unit, and/or for identifying whether a user corresponds to one of a (predetermined) set of different users.

By bio-impedance measurement unit is hereby meant a functional unit including the plurality of measurement electrodes and the measurement circuit, wherein bio-impedance measurements on the user is enabled by means of the measurement electrodes and the measurement circuit.

By the bio-impedance measurement unit being adapted to be worn on a body part of the user, measurements may be made for user identification without active control by the user, once the bio-impedance measurement unit has been attached on the body part. Advantageous examples of body parts include a wrist, an ankle, an arm portion, a leg portion or a chest portion of the user.

The bio-impedance measurement unit may be supported by a wearable adapted to be worn on a body part of the user. Advantageous examples of body parts include a wrist, an ankle, an arm portion, a leg portion or a chest portion of the user.

The wearable may advantageously be in the form of a strap adapted to be worn about a body part of the user. The strap may advantageously be a wrist band, an ankle strap, an arm strap, a leg strap or a chest strap.

The measurement electrodes may be distributed along the wearable such that the measurement electrodes are distributed over an area of the body part on which the wearable is adapted to be worn.

By "the measurement circuit" is hereby meant any circuitry being configured to measure an impedance in portions of the user via the different groups of measurement electrodes.

Advantageously, the measurement circuit may be adapted to perform bio-impedance measurements via groups including four measurement electrodes. A measurement via four measurement electrodes may be referred to as a tetrapolar measurement. Such a configuration enables reliable and accurate impedance measurements, with reduced sensitivity to effects such as a contact or coupling impedance between measurement electrodes and the skin of the user.

The measurement circuit may alternatively be adapted to perform bio-impedance measurements via groups including only two measurement electrodes. A measurement via two measurement electrodes may be referred to as a bipolar measurement. Such a configuration enables impedance measurements to be performed in a plurality of different portions of the user via a smaller amount of measurement electrodes compared to a measurement circuit configured for tetrapolar measurements.

The plurality of measurement electrodes may be any type of electrodes suitable for electrical measurements, in particular impedance measurements, on the user.

Advantageously, the plurality of measurement electrodes may be a plurality of skin electrodes adapted to be arranged in contact with a respective skin portion of the user.

According to another advantageous implementation, the plurality of measurement electrodes may be a plurality of electrodes adapted to be only capacitively coupled to a respective portion of the user.

The measurement circuit may be adapted to measure an impedance in each frequency channel of a set of a plurality of frequency channels. The set of frequency channels may advantageously be the same for each electrode group. This may facilitate data analysis and may ensure that an impedance is measured in a same manner via each group of electrodes.

By storage unit is hereby meant any unit being adapted to store each measured impedance (for instance by storing a representation of each measured impedance). The storage may be volatile or a non-volatile.

By identification unit is hereby meant any unit implementing functionality or logic allowing performing of a comparison based on the measurement data set and the reference data set. Various types of comparisons are possible as will be further set out below.

In case the system includes a wearable, the storage unit and, optionally, the identification unit may be supported by the wearable.

The identification unit may however also be arranged remote from bio-impedance measurement unit, wherein the bio-impedance measurement unit may be adapted to wirelessly transmit the measurement data set to the identification unit. The system may include a transmitter operatively coupled to the bio-impedance measurement unit. The system may include a receiver operatively coupled to the identification unit.

The identification unit may (regardless if being supported by the wearable or being arranged remote from the bio-impedance measurement unit) be adapted to, in response to receiving the measurement data set perform a comparison based on the measurement data set and the reference data set.

According to one embodiment the storage unit is adapted to store each measured impedance in a measurement array, and wherein the identification unit is adapted to compare the measurement data set to the reference data set by determining a difference between the measurement array and a reference array storing the reference data set. Thereby it is possible to determine a degree of similarity between the measurement array and the reference array.

The measurement array may include, for each group of measurement electrodes and for each frequency channel of said set of frequency channels, a storage position for storing a representation of a measured impedance, and wherein the reference array includes a storage position corresponding to each storage position of said measurement array. The measured impedances may hence be stored in an array data structure including a plurality of storage positions in an orderly fashion facilitating further analysis. By storage position is hereby meant a data element.

The identification unit may be adapted to determine the difference between the measurement array and the reference array by calculating a difference between a measured impedance and a reference impedance for each storage position of the measurement array and the reference array. Calculating, element-wise, a difference between the measurement array and the reference array enables a computationally efficient and easy implementable comparison of the measurement array and the reference array.

The identification unit may be further adapted to, based on the calculated differences, determine a distance between the measurement array and the reference array. By distance is hereby meant a scalar representing a distance between two points. In the present embodiment, the two points are determined by the measurement array and the reference array, respectively, wherein the storage positions of the arrays represent the coordinates of the points.

The identification unit may be adapted to provide output data based on the comparison.

The output data may indicate a result of the comparison. The output data may indicate whether the measurement data set matches the reference data set. The output data may indicate a difference or distance between the measurement array and the reference array. The identification unit may additionally or alternatively be adapted to provide output data including a key associated with the reference data set in response to the measurement data set matching the reference data set to a predetermined degree. The key may be a code or identifier. The key be a predetermined unique code associated with the reference data set, and hence associated with the user associated with the reference data set. The predetermined degree of matching may be a minimum degree of similarity between the measurement array and the reference array. A degree of matching meeting (or exceeding) the predetermined degree of matching may indicate that the user of the wearable is a valid user of the wearable and/or of another device or service in communication with the identification unit, or that the user of the wearable corresponds to one of a (predetermined) set of different users. A degree of matching lower than the predetermined degree of matching may indicate that the user of the wearable is not a valid user of the wearable and/or of another device or service in communication with the identification unit, or that the user of the wearable does not correspond to one of a (predetermined) set of different users.

According to one embodiment the measurement circuit is adapted to, prior to performing an impedance measurement via a group of measurement electrodes, determine whether an electrode of said group of measurement electrodes is in contact with or within a predetermined distance from a skin portion of the user.

This embodiment enables accurate bio-impedance measurements since it may be tested, prior to the impedance measurement, whether the group of measurement electrodes have no or a poor electric contact (i.e. galvanic and/or capacitive contact) with the skin of the user. An impedance measurement via the group of skin electrodes may then be avoided or skipped in response the measurement circuit determining that the electrode is not in contact with or is beyond a predetermined distance from the skin portion.

The plurality of measurement electrodes may be skin electrodes and wherein the measurement circuit may be adapted to determine whether said measurement electrode of said group is in contact with the skin portion by measuring a contact impedance between the skin portion and said measurement electrode or between the skin portion and a skin electrode adjacent to said measurement electrode. The skin electrode may form part of the group of skin electrodes. Alternatively, the skin electrode may be separate from the group of skin electrodes.

The plurality of measurement electrodes may be contact-less electrodes adapted to be (only) capacitively coupled to a respective skin portion of the user, wherein the measurement circuit may be adapted to determine whether said measurement electrode of said group is within the predetermined distance from the skin portion based on a capacitive impedance between said electrode and the skin portion. The measurement circuit may measure the capacitive impedance between said electrode and the skin portion and determine whether the measured capacitive impedance is greater than a predetermined capacitive impedance threshold.

According to one embodiment the system further comprises a wearable adapted to be worn about a body part of the user and supporting said plurality of measurement electrodes and the measurement circuit,
wherein the wearable comprises a first end portion and a second end portion, the first end portion comprising a first attachment member and the second end portion comprising a plurality of second attachment members being distributed along a longitudinal direction of the wearable, wherein the first attachment member is adapted to attach to any one of said plurality of second attachment members,
wherein, when the first attachment member is attached to any one of said plurality of second attachment members, a circuit path, extending along the wearable and having an electrical characteristic which is unique for said one of said plurality of second attachment members, is closed.

When the first attachment member is attached to any one of the plurality of second attachment members a closed loop wearable may be formed.

The unique electrical characteristic associated with each possible circuit path enables detection of which one of the second attachment members the first attachment member is attached to. By the second attachment members being distributed in a longitudinal direction of the wearable, the electrical characteristic may provide information of a circumference of the wearable, and hence the body part about which the wearable is worn.

The system may further comprise a wrist size determination unit adapted to output size data representative of a circumference of said body part, based on the electrical characteristic of said circuit. The biometric formed by the circumference of the body part of the user may be used for the purpose of identification of the user.

The storage unit may be adapted to store the size data in the measurement data set. Accordingly, the identification unit may perform a comparison based on the measurement data set and the reference data set, wherein the measurement data set includes a combination of measured impedances and the size data.

Brief description of the drawings

The above, as well as additional objects, features and advantages of the present inventive concept, will be better understood through the following illustrative and non-limiting detailed description of preferred embodiments of the present inventive concept, with reference to the appended drawings. In the drawings like reference numerals will be used for like elements unless stated otherwise.

Fig. 1 is a schematic block diagram of a biometric identification system.
Fig. 2 is a schematic illustration of a wrist band.
Fig. 3 is a schematic illustration of a wrist band with skin electrodes.
Fig. 4 is a schematic illustration of a wrist band with contact-less electrodes.

Detailed description

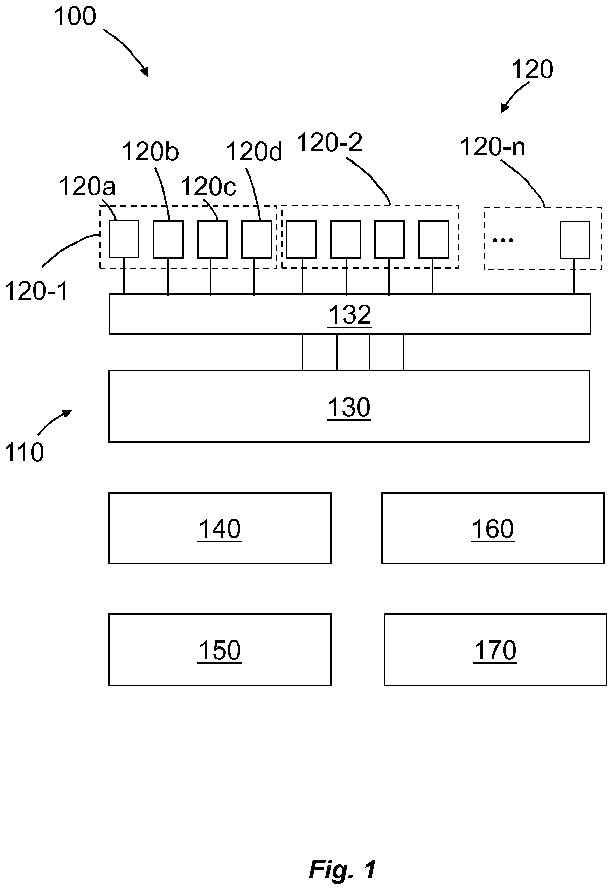

Fig. 1 illustrates a biometric identification system 100, hereinafter referred to as the system 100. The system 100 comprises a bio-impedance measurement unit 110 adapted to be worn by a (not shown) user. The bio-impedance measurement unit 110 (hereinafter referred to as the measurement unit 110) includes a plurality of measurement electrodes, commonly referenced 120. The measurement unit 110 includes an impedance measurement circuit 130 (hereinafter referred to as the measurement circuit 130) adapted to perform bio-impedance measurements on the user via a plurality of different groups 120-1, 120-2, ... 120-n of the plurality of measurement electrodes 120. The measurement circuit 130 is adapted to, for each group of measurement electrodes 120-1, ..., 120-n, measure an impedance $Z_{i,f}$ in each frequency channel f of a set of frequency channels F. The system 100 further comprises a storage unit 140 adapted to store each measured impedance $Z_{i,f}$ in a measurement data set M. The system 100 further comprises an identification unit 150 adapted to perform a comparison based on the measurement data set M and a reference data set R.
The system 100 may be used in various applications including for instance for providing identity credentials, for security control or access clearance at a facility or house or to a vehicle, identification for secure payments, device access to electronic devices such as desktop computers, laptops or other portable electronics such as tablet computing devices and mobile phones.
The measurement circuit 130 is adapted to measure a respective impedance at each one of a plurality of portions of a body part of the user via a respective group of measurement electrodes 120-1, ... , 120-n. As will be further described below the measurement electrodes 120 may be worn about a body part wherein the groups of measurement electrodes 120-1, ..., 120-n are circumferentially distributed about the body part.
Each group 120-1, ..., 120-n may be referred to as an impedance measurement channel. The number n of groups or, correspondingly, impedance measurement channels is an implementation choice and may be at least two, at least three, at least four or at least five. Preferably, the number n of impedance measurement channels is at least 10, more preferably at least 20 and even more preferably at least 40.
The measurement electrodes 120 are connected to the measurement circuit 130 via a switching circuit 132 adapted to selectively connect groups of measurement electrodes to the measurement circuit 130. The measurement circuit 130 is adapted to measure an impedance via the electrode group to which the impedance measurement circuit 130 is currently connected. The switching circuit 132 may be adapted to control which impedance measurement channel should be used at each instant. The switching circuit 132 may include a set of switches, such as relays or transistor-based switches for selectively connecting input/output terminals of the measurement circuit 130 to the measurement electrodes 120.
The functions and logic controlling the operation of the measurement circuit 130 and the switching circuit 132 may be implemented in a respective or in a common integrated circuit, an application specific integrated circuit (ASIC) or a field-programmable gate array (FGPA).
In the illustrated system 100, each group of measurement electrodes 120-1, 120-2, ..., 120-n includes four measurement electrodes 120a-d. The electrode groups 120-1, ..., 120-n are shown to include distinct, nonoverlapping, subsets of the measurement electrodes 120. It is also possible to configure the electrode groups to have electrodes in common such that the electrode groups overlap. As an example, a first electrode group may include a first through fourth measurement electrode (e.g. 120a-120d in Fig. 1) and a second electrode group may include the second through fourth measurement electrode (e.g. 120b-120d in Fig. 1) and a further fifth measurement electrode adjacent to the fourth measurement electrode (e.g. the un-numbered electrode next to the electrode 120d in Fig. 1). It is also possible to configure electrode groups with a smaller number of common measurement electrodes such as only two or only one.
The measurement electrodes 120 may be skin electrodes adapted to be arranged in electrical and physical contact with a respective skin portion of the user. The measurement circuit 130 may be adapted to perform a tetra-polar impedance measurement via the electrode groups 120-1, ..., 120-n. An impedance measurement will now be described in connection with the electrode group 120-1. An impedance measurement may be performed in a corresponding manner via the further measurement electrode groups 120-2, ..., 120-n. The measurement circuit 130 may be adapted to measure an impedance via the electrode group 120-1 by transmitting a test current from the electrode 120a of the group 120-1, into the user and to the electrode 120d of the group 120-1. The measurement circuit 130 may be adapted to, while the test current propagates between the electrodes 120a and 120d, measure a voltage between the electrodes 120b and 120c of the group 120-1.

A test current may for instance be generated by a signal generator of the impedance measurement circuit 130. The signal generator may include a controlled current source adapted to transmit an AC test current of a predetermined amplitude and frequency between the electrodes 120a and 120d. The amplitude of the test current may for example be in the range of 10 $\mu$A to 1 mA. The test current may be generated to present a frequency in accordance with a current frequency channel selected from the set of frequency channels F. The set of frequency channels F may for instance cover, or cover a sub-range of, 1 kHz to 1 MHz. Each frequency channel f may be a single predetermined frequency channel or cover a relatively narrow frequency band.

The absolute value of the impedance may be estimated from the ratio between the amplitude of the measured voltage and the amplitude of the test current. A phase of the impedance may be estimated from a phase difference between the test current and the measured voltage. The measurement circuit 130 may include an analog-to-digital converter for sampling and digitizing the test current transmitted between the first and the second electrode and the resulting voltage between the third and the fourth electrode. The measurement circuit 130 may thus calculate the absolute value of the body impedance and the phase of the body impedance in a digital domain.

As an alternative to the tetra-polar impedance measurement configuration, the system 100 may include groups of measurement electrodes 120-1, ..., 120-n including only two skin electrodes. The measurement circuit 130 may accordingly be adapted to perform a bi-polar impedance measurement via electrode groups 120-1, ..., 120-n including only two skin electrodes. A bi-polar impedance measurement may be implemented in a manner corresponding to the above-described tetra-polar measurement however with the difference that in a measurement via an electrode group, the test current and the voltage measurement is performed via a same pair of electrodes.

As an alternative to skin electrodes, the measurement electrodes 120 may be contact-less measurement electrodes 120, adapted to only be capacitively coupled to the user. Either tetra-polar or bi-polar impedance measurements are possible. An impedance measurement using contact-less measurement electrodes 120 may be implemented in a manner corresponding to the above however with the difference that no test current will be injected into the user but rather the test current will by capacitive coupling induce a varying voltage signal in the user, which may be measured by the measurement circuit 130.

The storage unit 140 shown in Fig. 1 is adapted to store each measured impedance in a storage structure in the form of a measurement array. The storage unit 140 may include a volatile memory such as a volatile random access memory (RAM), or a non-volatile memory such as a flash-based memory. The label M will in the following be used to refer to both the measurement data set and the measurement array. The measurement array M includes, for each group of measurement electrodes 120-1, ..., 120-n, and for each frequency channel f, a storage position for storing a representation of a measured impedance. The measurement array may be in the form a one-dimensional data array or vector.

As an example, the measurement array M may have a structure of

$$[Z_{\text{group 1},f=ch\#1}; Z_{\text{group 2},f=ch\#1}; \ldots ; Z_{\text{group n},f=ch\#1};$$
$$Z_{\text{group 1},f=ch\#2}; Z_{\text{group 2},f=ch\#2}; \ldots ; Z_{\text{group n},f=ch\#2};$$
$$\ldots$$
$$Z_{\text{group 1},f=ch\#k}; Z_{\text{group 2},f=ch\#k}; \ldots ; Z_{\text{group n},f=ch\#k}]$$

where each element denotes the impedance measurement via measurement electrode group i of the plurality of measurement electrodes 120, at frequency channel #j of the set of frequency channels F.

The storage unit 140 may further store the reference data set R in the form of a reference array including a storage position corresponding to each storage position of the measurement array. The label R will in the following be used to refer to both the reference data set and the reference array.

As an example, the reference array M may have a structure of

$$[R_{\text{group 1,f=ch\#1}}; R_{\text{group 2,f=ch\#1}}; \ldots ; R_{\text{group n,f=ch\#1}};$$

$$R_{\text{group 1,f=ch\#2}}; R_{\text{group 2,f=ch\#2}}; \ldots ; R_{\text{group n,f=ch\#2}};$$

$$\ldots$$

$$R_{\text{group 1,f=ch\#k}}; R_{\text{group 2,f=ch\#k}}; \ldots ; R_{\text{group n,f=ch\#k}}]$$

where each element denotes a reference impedance for the electrode group i of the plurality of measurement electrodes 120, at frequency channel #j of the set of frequency channels F.

The reference data set R may be a predetermined data set, established by performing bio-impedance measurements on a user with the system 100 one or more times and storing a representation of the measurements as the reference data set R. In case of plural measurements, an average of the measurements may be stored as the reference data set R.

The identification unit 150 is adapted to compare the measurement data set M to the reference data set R. The identification unit 150 may be implemented in an integrated circuit, an ASIC or an FPGA. The identification unit 150 may be implemented in a same integrated circuit, ASIC or FPGA (as the case may be) as the measurement unit 130.

The identification unit 150 is adapted to determine a difference between the measurement array M and the reference array R. The identification unit 150 is adapted to determine the difference by calculating a difference between a measured impedance and a reference impedance stored at each storage position of the measurement array and the reference array.

With reference to the above example structure of the measurement array M, the identification unit 150 may accordingly be adapted to calculate:

$$[Z_{\text{group 1,f=ch\#1}} - R_{\text{group 1,f=ch\#1}}; Z_{\text{group 2,f=ch\#1}} - R_{\text{group 2,f=ch\#1}}; \ldots ]$$

Based on this calculation the identification unit 150 may further be adapted to calculate a distance, e.g. in Euclidean sense, between the measurement array M and the reference array R. It should be noted that this represents only one possible way calculating a distance or degree of similarity between the measurement data set and the reference data set and that other calculations and norms also may be used.

The identification unit 150 is adapted to provide output data indicating a result of the comparison. The output data may include a degree of similarity, or a distance, between the measurement data set M and the reference data set R. Alternatively, the output data may be a binary output indicating that the measurement data set M matched the reference data set R (e.g. by a "1") or that there was no match (e.g. by a "0").

Alternatively or additionally, the identification unit 150 may be adapted to provide output data including a key associated with the reference data set in response to the measurement data set matching the reference data set. The key may be a code or identifier. The key be a predetermined unique code associated with the reference data set R, and hence associated with the user associated with the reference data set R. The key may be stored by the storage unit 140 and retrieved by the identification unit 150.

The identification unit 150 may be adapted to compare the measurement data set with each one of a plurality of reference data sets $\{R_x\}$ associated with each one of a plurality of different users. The identification unit 150 may in this case be adapted to provide output data including the key $k_x$ associated with the reference data set $R_x$ which presented a match or the smallest difference with or smallest distance to the measurement data set M. The storage unit 140 may store a reference data set $R_x$ and a key $k_x$ for each one of a plurality of different users.

The system 100 may include a wireless transceiver 160 adapted to communicate the output data provided by the identification unit 150 directly to a remote device, requiring a user identification, or to a remote device which may use the output data to extract or generate a (further) key which in turn may be used for identifying the user with respect to a service or device. The wireless transceiver may be a Bluetooth LE transceiver, an NFC transceiver, a WLAN transceiver or implement any other conventional type of wireless communications technology. The system 100 is however not limited to a wireless communication interface for communicating the output data. The output data of the identification unit 150 may alternatively be communicated to a receiving device via a wired communication interface, such as a USB-interface.

In use of the system 100, the switching circuit 132 may connect the measurement circuit 130 to one electrode group at a time. An active impedance measurement channel may thus be changed in a "time-division manner" wherein the measurements circuit 130 may measure an impedance via only one electrode group at a time. For each impedance measurement channel, the measurement circuit 130 may measure an impedance in each frequency channel f of the total set of frequency channels F. Each measurement may be stored at a corresponding storage position of the measurement array M, as set out above. The measurement circuit 130 may measure the resulting impedance for one frequency channel f at a time. The switching circuit 132 may thereafter cycle through all remaining impedance measurement channels such that an impedance is measured in each frequency channel f via each electrode group 120-1, ..., 120-n.

Once the impedance measurements have been completed, the identification unit 150 may compare the measurement array M to the reference array R (or to each one of the one or more reference arrays $R_x$) and provide output data (e.g. for communication by the wireless transceiver 160).

In a configuration where the measurement electrodes 120 are skin electrodes, the measurement circuit 130 may be adapted to, prior to performing an impedance measurement via a group of skin electrodes, such as group 120-1, determine, by measuring a contact impedance between one or more of the skin electrodes of the group 120-1 with a respective skin portion, whether the one or more skin electrodes of the group 120-1 is in contact with the skin. For instance, the measurement circuit 130 may be adapted to, prior to performing an impedance measurement via a group of skin electrodes, such as group 120-1, determine, by measuring a contact impedance via a pair of measurement electrodes 120a, 120b of the group 120-a, whether the measurement electrodes of the group 120-1 are in contact with a skin portion. In response the measured impedance falling below a predetermined threshold, the measurement circuit 130 may determine that the skin electrodes 120, 120b are in contact with a respective skin portion.

Based on this determination, it may be assumed that the other measurement electrodes of the group 120-1 also are in contact with a skin portion, and thus that a subsequent impedance measurement via the group 120-1 therefore is a valid and reliable impedance measurement. Accordingly, the measurement circuit 130 may be adapted to proceed and perform an impedance measurement via the full group of skin electrodes 120-1, for subsequent storage by the storage unit 140 in the measurement data set. If not, the electrode group 120-1 to which the electrode 120a belongs may be skipped during the subsequent impedance measurement. The identification unit 150 may further be adapted to data exclude the reference values of the storage positions of the reference data set R corresponding to the omitted impedance measurements (in this case those via group 120-1) from the comparison with the measurement data set M.

Optionally, a corresponding impedance measurement may be performed via also the other two electrodes 120c, 120d of the group 120-1 before concluding whether a reliable impedance measurement via the full group of skin electrodes 120-1 may be performed. In response to also the impedance measured via these other two electrodes falling below a predetermined threshold, the measurement circuit 130 may be adapted to proceed and perform an impedance measurement via the full group of skin electrodes 120-1.

The above procedures may be repeated for each further group of electrodes of the system, e.g. group 120-2 and each further group up to group 120-n.

Instead of performing the "contact impedance" measurement via electrodes forming part of the group 120-1, the system 100 may include, for each group of electrodes 120-1, ..., 120-n, a separate dedicated pair of skin electrodes associated with a respective group. The pair of skin electrodes may be associated with the group by being arranged adjacent to, or in close vicinity to the group, preferably less than a few millimeters from any electrode of the associated group 120-1, ..., 120-n.

In a configuration where the measurement electrodes 120 are contact-less measurement electrodes, the measurement circuit 130 may be adapted to, prior to performing an impedance measurement via a group of measurement electrodes such as group 120-1, determine whether an electrode such as electrode 120a of the group 120-1 is within a predetermined distance from a skin portion of the user. The measurement circuit 130 may measure the capacitance between the electrode 120a and the skin portion and determine whether the measured capacitance is greater than a predetermined capacitance threshold. The predetermined capacitance threshold may be established based on prior knowledge of which threshold provides a sufficient likelihood for resulting in a reliable impedance measurement result. Such a measurement may be repeated for each measurement electrode of each group 120-1, ..., 120-n. If a capacitance measured by an electrode such as electrode 120a does not exceed the predetermined capacitance threshold, the electrode group 120-1 to which the electrode 120a belongs may be skipped during the subsequent impedance measurement.

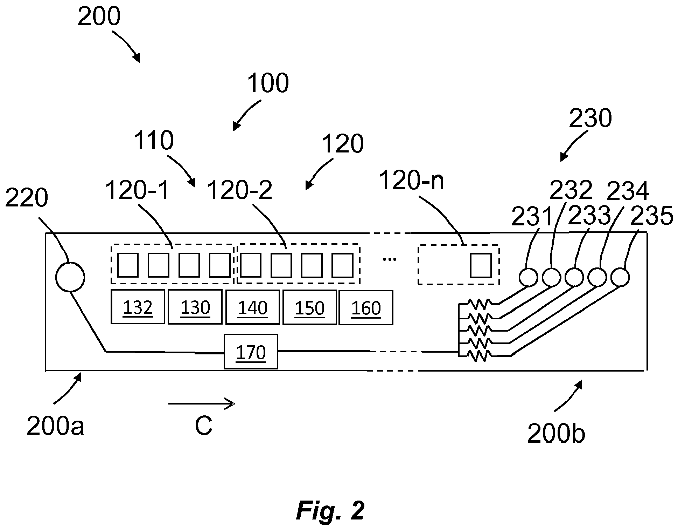

Fig. 2 is a schematic illustration of a wearable in the form of a wrist band 200 including the system 100 described in connection with Fig. 1. It should be noted that the wrist band 200 not is drawn to scale and that the measurement electrodes 120 as well as the various units (e.g. 110, 140, 150 etc.) are shown in a schematic sense to indicate that they may be supported by the wrist band 200, but are not shown in a manner corresponding to a real physical implementation.

The direction indicated by C is parallel to the longitudinal direction of the wrist band 200. The direction C hence corresponds to a circumferential direction of the wrist band 200 when the wrist band 200 is worn about the wrist of the user.

Generally, the wrist band 200 may be preformed to make a tight fit about the wrist when worn. The strap may include a flexible material, such as a woven material, a textile, a rubber or silicone material. The strap may alternatively include a (more) rigid material. The strap may for example include leather or a plastic material.

The wrist band 200 may be a wrist band for a watch, for instance a smart watch. The wrist band 200 may be a wrist band for a piece of jewelry such as a bracelet. The wrist band 200 may also be a dedicated bio-impedance measurement wrist band. Wearables suitable to be worn about other body parts such as an ankle, an arm portion, a leg portion or a chest portion, may also be provided in the form of a band or strap and have a configuration corresponding to the illustrate wrist band 200.

The wrist band 200 may include connection paths for interconnecting the various structures and units of supported by the wrist band 200, such as between the measurement electrodes 120 and the switching circuit 132. The connection paths may for instance be formed by conducting wires or by conducting paths printed on a flexible substrate (such as a thin plastic foil) and may be incorporated in a strap of the wrist wear 110.

The measurement circuit 130, the storage unit 140, the identification unit 150 and the wireless transceiver 160 may be implemented in respective or in a common integrated circuit, an application specific integrated circuit (ASIC) or a field-programmable gate array (FGPA) integrated in or otherwise supported by the wrist band 200. System-on-chip (SoC) implementations are also possible. The wrist band 200 may further support one or more power sources, such as batteries, for powering the system 100.

Although in Fig. 2 the measurement electrodes 120 are arranged to form a single line of aligned electrodes, other configurations are also possible such as an arrangement of electrodes in two rows.

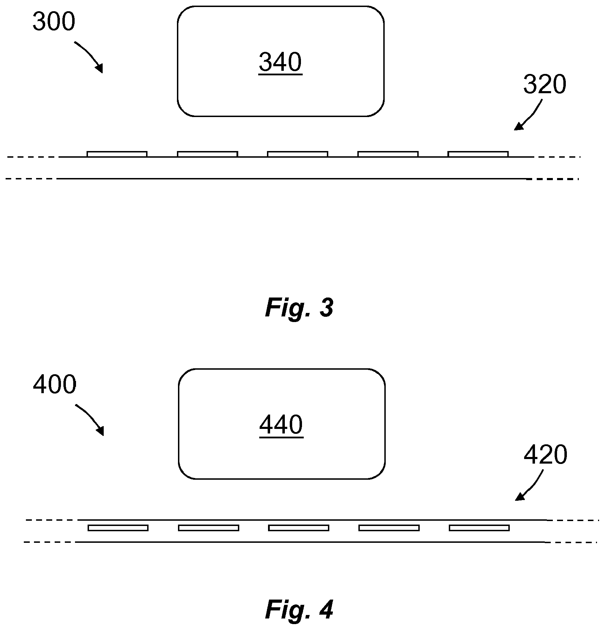

If the measurement electrodes 120 are provided in the form of skin electrodes, each skin electrode may include a separate conductive portion arranged on a surface of the wrist band 200 adapted to face the skin of the wearer. Such a configuration is schematically illustrated in Fig. 3 showing a wrist band 300 supporting, on a surface intended to face a wrist 340 of the user, skin electrodes 320 (corresponding to the measurement electrodes 120). When the wrist band 300 is worn about the wrist 340, the skin electrodes 320 will accordingly be arranged in physical and electrical contact with the skin of the wrist 340. Each conductive portion may include a conductive material such as stainless steel, copper, aluminum, gold, silver, silver-chloride or carbon, to name a few. The conductive material may be arranged as a thin layer on the surface of the wrist band 300. A thin layer may for instance be glued to the surface of the wrist band 300, or attached by mechanical attachment means. According to further options a conductive portion may be formed by a patch of conductive ink deposited on the surface of the wrist band 300, or by a conductive yarn incorporated into the surface of the wrist band 300 (e.g. interwoven with a strap of a woven material).

If the measurement electrodes 120 are provided in the form of contact-less measurement electrodes, adapted to only be capacitively coupled to the user, each electrode may include a separate conductive portion integrated inside the wrist band 200. Such a configuration is schematically illustrated in Fig. 4 showing a wrist band 400 including contact-less electrodes 420 (corresponding to the measurement electrodes 120) integrated inside the wrist band 400. When the wrist band 400 is worn about the wrist 440, the electrodes 420 will accordingly be separated from direct contact with the skin of the wrist 440 by a thickness portion of the wrist band 400 and only be capacitively coupled to the skin of the wrist 440. The conductive portion may include any of the conductive materials discussed above in connection with Fig. 3.

A visual marker (e.g. a line, a dot or other graphical shape) may be provided on the wrist band 200 to allow the user to wear the wrist band 200 with a consistent rotation in relation to the wrist through-out and between occasions of use. Especially, this may facilitate wearing the wrist band 200 with a same orientation as when the reference data set R was established.

With reference to Fig. 2, the wrist band 200 comprises a first end portion 200a and a second end portion 200b. The first end portion 200a comprises a first attachment member 220. The second end portion 200b comprises a plurality of second attachment members 230 being distributed along the longitudinal direction C of the wrist band 200. The first attachment member 220 is adapted to attach to any one of said plurality of second attachment members 230. The first attachment member 220 may be a pin and the second attachment members 230 may be correspondingly shaped pin-holes. Alternatively, the first attachment member 220 may be a first Velcro-part and the second attachment members 230 may be corresponding second Velcro-parts. Alternatively, the first attachment member 220 may be a first magnetic-part and the second attachment members 230 may be corresponding second magnetic parts. In Fig. 2, the wrist band 200 includes five second attachment members 231-235. This is however merely an example and the wrist band 200 may include fewer or more second attachment members 230.

A plurality of circuit paths are arranged to extend along the wrist band 200. As shown, each circuit path has a first terminal at the first attachment member 220 and a respective second terminal at each one of the second attachment members 230. The attachment members 220, 230 and the terminals are so arranged that a given one of the circuit paths are closed when the first attachment member 220 is attached to any one of the second attachment members 230. When the first attachment member 220 is attached to a second attachment member 230, the first terminal and the second terminal are brought into electrical contact wherein a closed circuit path is formed.

Each circuit path is arranged to present an electrical characteristic which is unique for said one of said plurality of second attachment members, is closed. For instance, each circuit path may as shown have a resistance, or more generally an impedance, which is unique for said circuit path. For instance the circuit path between 220 and 231 may present a first resistance or impedance; the circuit path between 220 and 232 may present a second resistance or impedance different from the first; the circuit path between 220 and 233 may present a third resistance or impedance different from the first and second, etcetera.

The system 100 may include a wrist size determination unit 170, supported by the wrist band 200, and being adapted to output size data representative of a circumference of the body part, based on the electrical characteristic of the circuit path. The wrist size determination unit 170 may measure the resistance or impedance of the circuit path and output size data based on the measured resistance. For instance, a first resistance or impedance may be associated with first size data, a second resistance or impedance may be associated with second size data, etcetera. The association between resistance / impedance and size data may for instance be established in a look-up-table of the wrist size determination unit 170. The wrist size determination unit 170 may retrieve the appropriate size data from the look-up-table and provide it as an output. The storage unit 140 may be adapted to store the output size data in the measurement data set M, wherein the size data, in conjunction with the impedance measurements, may be used as an extended biometric.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

1.  A biometric identification system (100) comprising:

    a bio-impedance measurement unit (110) adapted to be worn by a user, the bio-impedance measurement unit (110) including:

       a plurality of measurement electrodes (120);
       a measurement circuit (130) adapted to perform bio-impedance measurements on the user via a plurality of different groups (120-1, 120-2, 120-n) of said plurality of measurement electrodes (120), each of said groups including at least two measurement electrodes,
       wherein the measurement circuit (130), for each group of measurement electrodes (120-1, 120-2, 120-n), is adapted to measure an impedance in each frequency channel of a set of frequency channels,

    the system (100) further comprising:

       a storage unit (140) adapted to store each measured impedance in a measurement data set, and
       an identification unit (150) adapted to perform a comparison based on the measurement data set and a reference data set.

2.  The system of claim 1, wherein the storage unit (140) is adapted to store each measured impedance in a measurement array, and wherein the identification unit (150) is adapted to compare the measurement data set to the reference data set by determining a difference between the measurement array and a reference array storing the reference data set.

3.  The system of claim 2, wherein the measurement array includes, for each group of measurement electrodes and for each frequency channel, a storage position for storing a representation of a measured impedance, and wherein the reference array includes a storage position corresponding to each storage position of said measurement array.

4.  The system of claim 3, wherein the identification unit (150) is adapted to determine the difference by calculating a difference between a measured impedance and a reference impedance for each storage position of the measurement array and the reference array.

5.  The system of any of the preceding claims, wherein the identification unit (150) is adapted to provide output data based on the comparison.

6.  The system of any of the preceding claims, wherein the measurement circuit (130) is adapted to, prior to performing an impedance measurement via a group of measurement electrodes (120-1, 120-2, 120-n), determine whether an electrode of said group of measurement electrodes is in contact with or within a predetermined distance from a skin portion of the user.

7.  The system of claim 6, wherein said plurality of measurement electrodes are skin electrodes and wherein the measurement circuit (130) is adapted to determine whether said measurement electrode of said group (120-1, 120-2, 120-n) is in contact with the skin portion by measuring a contact impedance between the skin portion and said measurement electrode or between the skin portion and a skin electrode adjacent to said measurement electrode.

8. The system of claim 6, wherein the plurality of measurement electrodes are contact-less electrodes adapted to be capacitively coupled to a respective skin portion of the user, wherein the measurement circuit (130) is adapted to determine whether said measurement electrode of said group (120-1, 120-2, 120-n) is within the predetermined distance from the skin portion based on a capacitive impedance between said electrode and the skin portion.

9. The system of any of the preceding claims, further comprising a wearable (200) adapted to be worn about a body part of the user and supporting said plurality of measurement electrodes (120) and the measurement circuit (130),

    wherein the wearable (200) comprises a first end portion (200a) and a second end portion (200b), the first end portion (200a) comprising a first attachment member (220) and the second end portion (200b) comprising a plurality of second attachment members (230) being distributed along a longitudinal direction of the wearable (200), wherein the first attachment member (220) is adapted to attach to any one of said plurality of second attachment members (230),
    wherein, when the first attachment member (220) is attached to any one of said plurality of second attachment members (230), a circuit path, extending along the wearable (200) and having an electrical characteristic which is unique for said one of said plurality of second attachment members (230), is closed.

10. The system of claim 9, further comprising a wrist size determination unit (170) adapted to output size data representative of a circumference of said body part, based on the electrical characteristic of said circuit path.

11. The system of claim 10, wherein said storage unit (140) is adapted to store said size data in the measurement data set.

12. The system of any of the preceding claims, including a wearable (200) adapted to be worn about a body part of a user, said wearable (200) supporting said plurality of measurement electrodes (120) and the measurement circuit (130).

13. The system of claim 12, wherein said wearable (200) supports the storage unit (140).

14. The system of claim 12 or 13, wherein said wearable supports the identification unit (150).

**Fig. 1**

Fig. 2

300

340

320

**Fig. 3**

400

440

420

**Fig. 4**

**Europäisches Patentamt**
**European Patent Office**
**Office européen des brevets**

# EUROPEAN SEARCH REPORT

Application Number

EP 17 17 6386

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/165450 A1 (HUNT ALEXANDER [SE] ET AL) 9 June 2016 (2016-06-09) | 1-5, 12-14 | INV.<br>G06F21/32<br>H04L29/06 |
| Y | * paragraph [0050] - paragraph [0052] *<br>* paragraph [0049] *<br>* paragraph [0034] *<br>* paragraph [0068] *<br>* paragraph [0084] *<br>* paragraph [0053] *<br>* paragraph [0054] *<br>* paragraph [0059] *<br>* paragraph [0064] *<br>* paragraph [0076] *<br>* paragraph [0067] *<br>* paragraph [0069] - paragraph [0072] *<br>* figures 6A-6C, 9, 10 * | 6-11 | ADD.<br>A61B5/053<br>A61B5/00<br>G06F21/34 |
| Y | US 2015/135310 A1 (LEE YONG JIN [US]) 14 May 2015 (2015-05-14)<br>* paragraph [0080] *<br>* paragraph [0030] *<br>* paragraph [0076] *<br>* figures 7,11 * | 6-8 | |
| Y | US 2016/267314 A1 (OHTSUKA TOSHIHIKO [JP] ET AL) 15 September 2016 (2016-09-15)<br>* paragraph [0180] - paragraph [0181] *<br>* paragraph [0182] *<br>* figure 13 * | 9-11 | TECHNICAL FIELDS SEARCHED (IPC)<br><br>A61B<br>G06F<br>H04L |
| A | US 2015/145676 A1 (ADHIKARI SURANJIT [US] ET AL) 28 May 2015 (2015-05-28)<br>* paragraph [0323] - paragraph [0326] *<br>* paragraph [0331] *<br>* paragraph [0300] - paragraph [0302] *<br>* figure 8 * | 9-11 | |

The present search report has been drawn up for all claims

| Place of search | Date of completion of the search | Examiner |
|---|---|---|
| Munich | 5 October 2017 | De la Hera, Germán |

EPO FORM 1503 03.82 (P04C01)

## ANNEX TO THE EUROPEAN SEARCH REPORT
## ON EUROPEAN PATENT APPLICATION NO.

EP 17 17 6386

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2017

| Patent document cited in search report | | Publication date | Patent family member(s) | | Publication date |
|---|---|---|---|---|---|
| US 2016165450 | A1 | 09-06-2016 | CN 107004078 A | | 01-08-2017 |
| | | | EP 3227814 A1 | | 11-10-2017 |
| | | | US 2016165450 A1 | | 09-06-2016 |
| | | | WO 2016089445 A1 | | 09-06-2016 |
| US 2015135310 | A1 | 14-05-2015 | US 2015135310 A1 | | 14-05-2015 |
| | | | WO 2015051253 A2 | | 09-04-2015 |
| US 2016267314 | A1 | 15-09-2016 | CN 105975901 A | | 28-09-2016 |
| | | | JP 2016167177 A | | 15-09-2016 |
| | | | US 2016267314 A1 | | 15-09-2016 |
| US 2015145676 | A1 | 28-05-2015 | NONE | | |

EPO FORM P0459

For more details about this annex : see Official Journal of the European Patent Office, No. 12/82